# EUROPEAN PATENT APPLICATION

(11) **EP 1 002 544 A1**
(43) Date of publication of application: **24.05.2000**
(21) Application number: 99122026.0
(22) Date of filing: 12.11.1999
(51) Int. Cl.: A61K 39/395, A61K 31/415

(54) **Anti-edema agent**

(30) Priority: 13.11.1998 JP 32401298
(71) Applicant: NIPPON ZOKI PHARMACEUTICAL CO., LTD., Chuo-ku, Osaka (JP)
(72) Inventor: Naiki, Mitsuru, Inst. of Bio-Active Science, Yashiro-cho, Katoh-gun, Hyogo (JP)
(74) Representative: HOFFMANN - EITLE

(57) **Abstract**

The present invention provides a pharmaceutical agent having a suppressive action against increase of vascular permeability of blood-brain barrier and also a suppressive action against edema formation, which show a low incidence of side effects and which contain histamine-added immunoglobulin as an effective component. Due the low side effects the agent of the present invention is very useful as anti-edema agent, a suppressive agent against edema formation and a suppressive agent against increase of vascular permeability of blood-brain barrier having high safety.

## Description

### Technical Field

The present invention relates to a novel drug containing histamine-added immunoglobulin as an effective component, more particularly, an anti-edema agent, a suppressive agent against edema formation and a suppressive agent against increase of vascular permeability of blood-brain barrier containing histamine-added immunoglobulin as an effective component.

### Prior Art

A complex of immunoglobulin and histamine has been known as a drug preparation, histamine-added immunoglobulin. It restores histamine fixing ability which is lowered in patients suffering from allergy and asthma, and so is used as an agent for nonspecific hyposensitizing therapy for bronchial asthma, allergic rhinitis, vasmotor rhinitis and allergic skin diseases such as urticaria, chronic eczema, atopic dermatitis, etc. Histamine-added immunoglobulin also exhibits a suppressive action against histamine liberation. It does not have any side effects exhibited by antihistamines and adrenocortical hormones used as symptomatic remedies. It has therefore been widely used as a pharmaceutical agent with high safety [See pages 463 and 464 of "Drugs in Japan, Ethical Drugs" (published in October 1996; edited by Japan Pharmaceutical Information Center; published by Yakugyo Jiho Co., Ltd.)].

In addition to said medical indications, it has been reported that histamine-added immunoglobulin has a suppressive action against hypereosinophilicity, immunomodulating action and the like, and the oral administration of histamine-added immunoglobulin expresses the same pharmacological activity as shown by usual hypodermic injection (Japanese Laid-Open Patent Publications Hei-7/53406, Hei-9/31311, etc.). However, in prior articles, there is no description regarding a suppressive action against edema formation and a suppressive action against increase of vascular permeability of blood-brain barrier of histamine-added immunoglobulin.

### Summary of the Invention

An object of the present invention is to provide a novel anti-edema agent, a suppressive agent against edema formation and a suppressive agent against increase of vascular permeability of blood-brain barrier, which have high safety with less side effects.

The present inventor has conducted an extensive investigation on said histamine-added immunoglobulin and found that it also has a suppressive action against edema and a suppressive action against the increase of vascular permeability of blood-brain barrier. The present invention provides a novel anti-edema agent, a suppressive agent against edema formation and a suppressive agent against increase of vascular permeability of blood-brain barrier containing histamine-added immunoglobulin as an effective component.

### Brief Explanation of Drawings

Figure 1 is a graph showing an example of a suppressive action of the agent of the present invention against increase of vascular permeability of blood-brain barrier accompanied with EAE.
Figure 2 is a graph showing an example of a suppressive action of the agent of the present invention against edema formation accompanied with EAE.

### Detailed Description of the Invention:

Histamine-added immunoglobulin, which is an effective component of the pharmaceutical agent of the present invention, is a complex of immunoglobulin and histamine, and can be prepared by mixing of an immunoglobulin component with a histamine component to obtain a substantially homogeneous mixture. In the case of an agent used for human being, it goes without saying that human immunoglobulin may be used as a material and such human immunoglobulin can be obtained from serum or placenta plasma by common methods. In order to secure the safety as a pharmaceutical agent, the standards which are usually stipulated for plasma fraction preparations are to be satisfied. For example, human plasma which is negative to HBs antigen, HCV antibody and HIV antibody is used and is further subjected to a heating treatment to avoid the danger of contamination of hepatitis virus and AIDS virus. The heat treatment is commonly used for inactivation of virus and, for example, a liquid-heat treatment at 60°C for 10 hours, a steam-heat treatment at 60°C for 10 hours, a dry-heat treatment at 65°C for 96 hours, etc. are usually conducted for fractionated plasma preparations.

In the case of application to animals other than human being, immunoglobulin may be prepared from an animal other than human being depending upon the species of the animal to be treated. Immunoglobulin has various classes such as IgG, IgA, IgM, etc. As an immunoglobulin component of the present invention, it can be used each of them, and also either solely or jointly together.

Free histamine and its pharmaceutically acceptable salts such as hydrochloride, phosphate and picrate may be used as a histamine component. Histamine dihydrochloride can be employed as a preferable one.

In the manufacture of the pharmaceutical agent of the present invention, it can be prepared, for example, by dissolving 1-200 mg, preferably 5-50 mg, of immunoglobulin and 0.01-2 µg, preferably 0.05-0.5 µg, of histamine component in a suitable solution such as physiological saline, distilled water, etc. with conventional means, mixing and stirring. It can be storable in freezing or as a freeze-drying form.

Histamine-added immunoglobulin of the present invention can be made into pharmaceutical preparations by a combination with a suitable pharmaceutical carriers or diluents. It can be made into various types of preparations by a common method and is able to be made into solid, semisolid, liquid or aerosol formulation for oral or parenteral administration. In preparing the agent, histamine-added immunoglobulin of the present invention can be used either solely or jointly together with other pharmaceutically active components.

In the case of injections, it is preferable to be made into a pharmaceutical composition as an isotonic solution using distilled water for injection or physiological saline solution. In its manufacture, additives such as auxiliary solubilizers, isotonizing agents, stabilizers, buffers, preservatives, etc. may be used in addition to histamine-added immunoglobulin. Examples of the applicable are citric acid, sodium benzoate, glycine, sodium sulfite, sodium bisulfite, sodium pyrosulfite, sodium thiosulfate, cysteine hydrochloride, phosphates, sodium ascorbate, sodium chloride, sodium bicarbonate, etc. Further, the agent of the present invention may be prepared as an injectable preparation which is dissolved upon actual use. Thus, the agent may be prepared in a dry state or a solution filled in vials or the like followed by a freeze-drying. In the manufacture of the dry preparation for injection, fillers such as glucose, mannitol and sorbitol may, if necessary, be added in addition to the above mentioned additives.

In the case of the preparations for oral administration, histamine-added immunoglobulin of the present invention solely or together with commonly used excipients such as a suitable additive (e.g. lactose, mannitol, corn starch, potato starch, etc.) is mixed with binders such as crystalline cellulose, cellulose derivatives, gum arabicum, corn starch, gelatin, etc., disintegrating agents such as corn starch, potato starch, carboxymethylcellulose potassium, etc., lubricating agents such as talc, magnesium stearate, etc. and others including bulking agents, moisturizing agents, buffers, preservatives, perfumes and the like to give tablets, diluted powders, granules or capsules. It is also possible, depending upon the type of the disease or the condition of the patient to prepare the pharmaceutical preparations which are other than those which were mentioned already and are suitable for the therapy such as, for example, inhalations, aerosol preparations, ointments, collyriums, suppositories, etc.

The preferred dose of histamine-added immunoglobulin of the present invention may vary depending upon the type of the disease, the condition of the patient, age or sex of the patient, form of the preparation, method for the administration, term for the administration, etc. and, in order to achieve a desired effect, 1-300 mg, preferably 5-150 mg may be usually given to common adults once or several times a week by hypodermic injection, although the present invention is not particularly limited to such dosage.

Preferred embodiments of the present invention are given as follows.
(1) An anti-edema and/or edema fomation suppressing agent containing histamine-added immunoglobulin as an effective component.
(2) A suppressive agent against increase of vascular permeability of blood-brain barrier containing histamine-added immunoglobulin as an effective component.
(3) An agent according to any of the above (1) to (2), wherein the ratio of immunoglobulin to histamine component is 1-200 mg of immunoglobulin component to 0.01-2 µg of histamine component, preferrably 5-50 mg to 0.05-0.5 µg, more preferrably 12.0 mg to 0.15 µg.
(4) An agent according to any of the above (1) to (3), in which immunoglobulin component is human immunoglobulin.
(5) An agent according to any of the above (1) to (4), in which histamine component is histamine dihydrochloride.
(6) An agent according to any of the above (1) to (5) formulated as an injectable preparation.

The following examples, which are illustrative only and not intended to limit the scope of the invention, describes the present invention more concretely.

### Examples

An example of the preparation of histamine-added immunoglobulin of the present invention is shown as follows. Rats were used as experimental animals in the following pharmacological tests and, accordingly, rat immunoglobulin was used in place of human immunoglobulin. Thus, for the pharmacological tests on rats, rat immunoglobulin and histamine dihydrochloride were dissolved in physiological saline solution by the following mixing ratios, stirred at room temperature for 2 hours, freeze-dried and upon use, dissolved by adding a physiological saline solution thereto for hypodermic administration.

| Product of the present invention | Amount of rat immunoglobulin | Amount of histamine. 2HCl |
|---|---|---|
| HG 50 | 5,3 mg | 0,10 µg |
| HG 75 | 12,0 mg | 0,15 µg |
| HG 90 | 28,8 mg | 0,30 µg |

Each of HG50, HG75 and HG90 products prepared above exhibited significant effects in all of the following pharmacological tests and, accordingly, only the results obtained for HG75 are given represntative thereof.

### Examples

The pharmacological tests were carried out as follows.

### 1) Animals

Lewis rats (6-10 weeks old, female, SPF) were purchased from Charles River, Japan. Rats were preliminarily maintained for 5 days in an animal room (22 ± 2°C room temperature and 55 ± 10% humidity) with artificial lightning for 12 hours from 8 a.m. to 8 p.m., and then healthy and normal rats were utilized for experiments.

### 2) Preparation of histamine-added rat immunoglobulin and administration thereof

Rat immunoglobulin prepared from normal rat serum (Cohn fractions II and III) and histamine dihydrochloride were dissolved in physiological saline in final concentrations of 26.6 mg/mL and 0.333 µg/mL respectively. The mixture was stirred for 2 hours and then utilized as HG75. HG75 was injected subcutaneously into the back of rat on every second day from immunizing day or cell-transferring day. To a control rat, physiological saline was injected as the same manner as HG75.

### 3) Method for inducing passive experimental allergic encephalomyelitis (pEAE)

The solution (400 µg/mL) of guinia pig myelin basic protein (GB-MBP 68-84) including synthetic peptide (MBP 68-84) corresponding the the encephalitogenic determinants was mixed with an equal volume of complete adjuvant (H37Ra) to give an emulsion. The emulsion (0.1 mL) was subcutaneously administered to the left hind foot pad of Lewis rat anesthetized with diethylether. The spleen cells were isolated from rats 10-14 days after the immunization and the cell suspension was prepared. Erythrocytes were removed by using the solution of 0.75% ammonium chloride and 17 mM Tris-HCl (pH = 7.65). After centrifugation, the spleen cells were suspended in RPMI-1640 medium and viable cells were counted by using 0.4% trypan blue solution. The cells were suspended at the concentration of 2 × 10⁶ cells/mL in the medium (RPMI-1640 containing 10 mM HEPES, 20 mM glutamine, 50 µg/mL penicillin, 50 U/mL streptomycin and 5 × 10⁻⁵M 2-mercaptoethanol) supplemented with 7% fetal calf serum. The cells (2 × l0⁷ cells/10 mL) were cultured in a culture dish at 37(C in an atmosphere of 5% CO₂ and 95% air in the presence of 2 µg/mL Concanavalin A. Cultured cells were collected and washed 3 times by centrifugation. The aggregated cells were removed by filtration, the cell concentration was determined by using 0.4% trypan blue. The cell concentration was adjust to 4 × 10⁷ cells/mL by physiological saline. Lewis rat was irradiated with 800 rad X-ray by the X-ray irradiator (MBR-1520R, Hitachi, Japan) and then the cells (2 × 10⁷ cells/0.5 mL) were transferred intravenously into tail vein.

### 4) Clinical Assessment of EAE

The clinical signs of the disease on tested rats were assessed by using clinical index to grade animals on indexes from 0 to 5 as follows. When the clinical sign of grade from 2 to 5 appeared partially, the value by subtracting 0.5 from each grade was adopted. The clinical signs were assessed by a blind method, namely, the treatment of rat such as with or without test drug was not clarified for the operator.

| Grade | Clinical Sign |
|---|---|
| 0 | Normal |
| 0.5 | Slight tail weakness |
| 0.75 | Moderate tail weakness |
| 1 | Disappearance of ability to hold tail up |
| 2 | Inactive |
| 3 | Paralysis of one hind leg |
| 4 | Paralysis of both hind legs |
| 5 | Paralysis of both hind legs accompanied by urinary incontinence |

### 5) Measurement of vascular permeability and edema

At a settled day after cell transfer, 2% evans blue solution (diluted with saline) was intravenously injected into rats (1 mL per 100g body weight). Two hours later, rats were perfused with 250 mL saline and then spinal cords were isolated. The wet weight of isolated spinal cords was measured. The spinal cords were dried at 90°C for 24 hours and the dry weight was measured. The water content was determined by deducting the dry weight from the wet weight.

To determine the amount of evans blue infiltrated into central nervous system, 0.5 mL of 1 N potassium hydroxide was added to spinal cords tissues and cultured at 37°C for 18 hours after well stirred. In addition, 4.5 mL of 0.6 N phosphoric acid-acetone buffer (5:13) was added into the mixture and well stirred again. After removal of precipitate from the mixture by filtration, absorbance (OD: 620 nm) of the filtered solution was measured. The standard solution of various concentrations added with from 0.3125-10 µg of evans blue were prepared to obtain the standard curve, and the amount of evans blue in each sample was calculated from the standard curve.

### 6) Statistical analysis

The amounts of evans blue in spinal cords and water contents were shown as mean ± S.E., and were analyzed with Scheffe's test (multiple comparison method) to obtain the significant difference compared to the control. The results are shown as below.

### 7) Progress of changes of vascular permeability accompanied with EAE

To clarify the participation of increase of vascular permeability of blood-brain barrier (BBB) in the onset of EAE, evans blue was administered intravenously from the cell transferred date and the amount of evans blue infiltrated into spinal cords within a settled time was measured. In the rats on the 3rd day after cell transfer (average clinical score: 0), the amount of evans blue infiltrated into spinal cords was not different from the control group (rats without cell transfer). On the 4th day after cell transfer (average clinical score: 0.7), the onset of EAE can be observed and the amount of evans blue infiltrated into spinal cords increased significantly (p<0.05) compared with the control group. On the 5th day after cell transfer (average clinical score: 3.0), the amount of evans blue infiltrated into spinal cords was the same level as observed on the 4th day and increased significantly (p<0.05) compared with the control group. However, no significant difference in the amount of evans blue compared to the control group was observed on the 7th day (average clinical score: 1.0) in which the onset of EAE were decreased and on the 11th day in which the appearance of EAE were disappeared.

### 8) Suppressive effect of the agent of the present invention on increase of vascular permeability accompanied with EAE

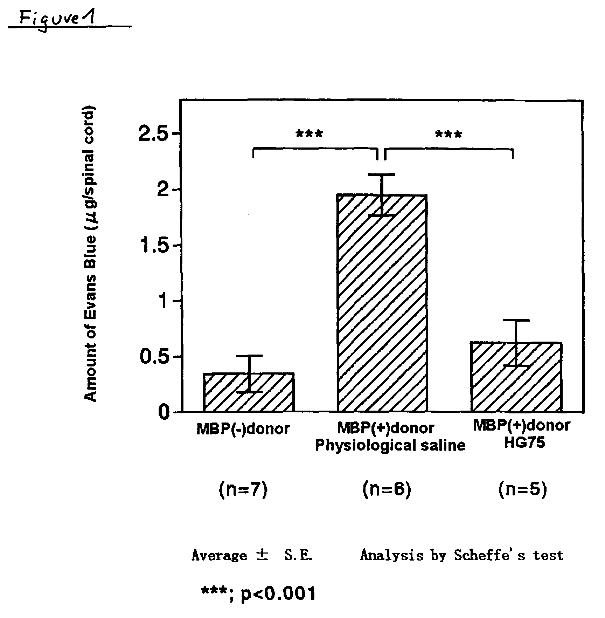

It was suggested that vascular permeability had some relationship with onset of pEAE, because the increase of vascular permeability of BBB was observed prior to the appearance of EAE. To study the effects of the present pharmaceutical agent on increase of vascular permeability, HG75 (150 mg/kg) was administered subcutaneously to the back of rat 3 times, every other day from the cell transferred date. To the rats 5 days after the cell transferred, 2% of evans blue solution was administered intravenously (0.1 mL/100 g). Two hours later, hemoperfusion from the rat heart using 400 mL of saline was conducted to remove evans blue from the peripheral blood. The whole spinal cords were isolated and the amount of infiltrated evans blue was measured. The example of the results was shown in Figure 1. As indicated in Figure 1, the amount of evans blue in the EAE inducted rats was increased to about 6 times as that of in the EAE non-inducted rats (a group without administration of MBP) and significant difference (p<0.001) was observed. On the other hand, the amount of evans blue in the rats to which the agent of the present invention was administered was the same level as that of in the EAE non-inducted rats and significant suppressive effect (p<0.001) was observed as compared to the control EAE rats.

### 9) Suppressive effect of the agent of this invention on edema formation accompanied with EAE

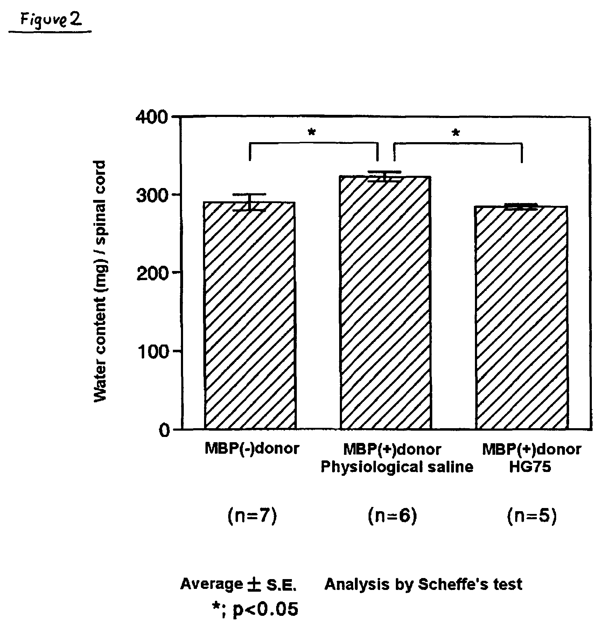

On the 5th day from the cell transferred date, the spinal cords were isolated and dried for 24 hours. Water content was calculated by deducting the dry weight of spinal cords from the wet weight of it. We studied the effect of the agent of this invention (150 mg/kg) by evaluation using said water content as an index. The example of the results was shown in Figure 2. As indicated in Figure 2, the water content in spinal cords in the EAE inducted rats was significantly increased (p<0.05) compared with the EAE non-inducted rats. On the other hand, the water content in spinal cords in the rats administered with the present pharmaceutical agent was decreased to the same level of that of in the EAE non-inducted rats and significant decreasing (p<0.05) was observed as compared with the control EAE rats.

### Effect of the Invention

As shown in Figure 1, the pharmaceutical agent of the present invention has a suppressive action against increase of vascular permeability of blood-brain barrier. It is also apparent from Figure 2, the agent of this invention has a suppressive action against edema formation. In the earliest stage of the formation of demyelination, it has been suggested that abnormality of blood-brain barrier is important. It has been clarified that blood-brain barrier attaches primary importance to cerebrovascular endothelial cells in the central nervous system. In the normal state, blood-brain barrier has very close conjugations and the number of small holes on endothelial cells is very small. According to such particular structure of endothelial cells different from other region, the transfer of substances in blood vessels to cerebral parenchyma is protected by blood-brain barrier. However, in edema regions, acute increase of vascular permeability of endothelium is observed. Therefore, the agent of the present invention is very useful as a suppressive agent against increase of vascular permeability of blood-brain barrier, an anti-edema agent and a suppressive agent against edema formation.

The low incidence of side effects has been well known regarding histamine-added immunoglobulin which is an active component of the agent of the present invention. Therefore, the agent of the present invention is very useful as an anti-edema agent, a suppressive agent against edema formation and a suppressive agent against increase of vascular permeability of blood-brain barrier having high safety.

## Claims

1. Use of histamine-added immunoglobulin as an effective component in the manufacture of a pharmaceutical composition effective in the formation supression and/or treatment of edema.

2. Use of histamine-added immunoglobulin as an effective component in the manufacture of a pharmaceutical composition effective in suppressing the increase of vascular permeability of the blood-brain barrier.

3. Use according to any of claims 1 to 2, wherein the ratio of immunoglobulin to histamine component is 1-200 mg of immunoglobulin component to 0.01-2 µg of histamine component.

4. Use according to claim 3, wherein the ratio is 5-50 mg of immunoglobulin component to 0.05-0.5 µg of histamine component.

5. Use according to claim 4, wherein the ratio is 12.0 mg of immunoglobulin component to 0.15 µg of histamine component.

6. Use according to any of claims 1 to 5, wherein the immunoglobulin component is human immunoglobulin.

7. Use according to any of claims 1 to 6, wherein the histamine component is histamine dihydrochloride.

8. Use according to any of claims 1 to 7, wherein the pharmaceutical composition is formulated as an injectable preparation.
